# EUROPEAN PATENT APPLICATION

(11) **EP 2 116 207 A1**
(43) Date of publication of application: **11.11.2009**
(21) Application number: 08008613.5
(22) Date of filing: 07.05.2008
(51) Int. Cl.: A61B 18/22

(54) **Diffusing tip**

(71) Applicant: Institut National De La Sante Et De La Recherche Medicale, 75013 Paris (FR)
(72) Inventor: The designation of the inventor has not yet been filed
(74) Representative: Sartorius, Jérome

(57) **Abstract**

A method for manufacturing a plug, comprising the flowing, in a tube (12), of a first fluid (F1) and a second fluid (F2), said second fluid (F2) being in contact, along an interface (I₁₂), with said first fluid (F1) so that the friction on the inside surface (14) of the tube (12) deforms this interface (I₁₂), the first and second fluids presenting different optical properties.

## Description

The invention concerns a method for manufacturing a diffuser tip, and more generally a method for manufacturing a plug which, in particular, may present a variable refractive index along its length.

The invention also concerns a diffuser tip manufactured according to a method of the invention.

### STATE OF THE ART

The laser induced thermotherapy (LITT) is used for therapeutic treatment in oncology. LITT destroys deep tumours by thermal effect while minimizing the impact on adjacent healthy tissues, using fibre optical based applicators. These applicators conventionally comprise a source of light, an optical fibre which proximal end is optically coupled to the source of light, and a diffuser tip optically coupled to the distal end of said optical fibre.

The diffuser tip is intended to control the light irradiation, so that a uniform, predictable dosage may be distributed. Indeed, the energy deposition surfaces in the irradiated tissues are very small, involving high irradiancies (kW/cm²). If not controlled, the rise in temperature around the applicator would rapidly lead to phenomena of charring, and a poor control of volume necrosis. The diffuser tip increases the energy deposition surface and therefore limits the temperature at the end of the applicator.

Examples of diffuser tips are disclosed in WO 93/25155, WO 00/79319, WO 99/11323, US 5,946,441, WO 98/11462 or in the article "A comparative optical analysis of cylindrical diffuser fibres for laser therapy using fluorescence imaging" by P.M. RIPLEY et al., in Lasers Med. Sci., 1999, 14:257-268, 1999, Springer-Verlag London Ltd..

An ideal diffuser tip should maintain a uniform irradiance both along its length and circumferentially, thus creating a symmetric homogenous cylinder of light.

As explained in the above-mentioned article of RIPLEY et al., different methods are available to manufacture diffuser tips. In particular, it is known that scattering particles, or "scattering centres", may be embedded in an optically transparent elastomer substrate. Modifying, stepwise or continuously, the concentration of scattering centres along the length of the diffuser enables an efficient control of the light irradiation.

For instance, WO 93/25155 discloses cylindrical diffuser tips comprising a cylindrical tip jacket containing a scattering layer.

In an embodiment, silicon cylindrical blocks containing various concentrations of scattering centres are moulded within the scattering layer along the axis of the diffuser tip.

In another embodiment, the diffuser tip comprises a conical transparent central core surrounded by a scattering layer. The diameter of the transparent central core of the diffuser tip decreases when approaching the distal end of the diffuser tip. Therefore, the laser light which exits radially from the diffuser tip encounters a greater number of scattering centres in the scattering layer before exiting the tip jacket. The intensity of the cylindrically diffused light along the axis of the diffuser advantageously varies more smoothly than with the previous embodiment, wherein light encounters stepwise concentration gradients of scattering centres as it enters the successive cylindrical blocks.

In a third embodiment, the diffuser tip comprises a silicon core disposed in a plastic tube and containing a variable concentration of scattering centres along the length of the diffuser tip. If the concentration increases exponentially along the diffuser tip, the intensity distribution of the cylindrically diffused light tends to be linear with a zero slope.

However, the manufacturing of the diffuser tips according to the state of the art is generally complex and expensive. In addition, these diffuser tips do not always provide a satisfactory uniformity in the intensity of exiting light along the length of the diffuser tip.

An object of the present invention is to provide a solution to at least one of these problems.

### SUMMARY OF THE INVENTION

The invention provides a method for manufacturing a plug, comprising the flowing, in a tube, of a first fluid and a second fluid, said second fluid being in contact, along an interface, with said first fluid, so that the friction on the inside surface of the tube deforms said interface, said first and second fluids preferably presenting different optical properties.

The deformation of said interface may be explained, in particular according to Navier-Stokes formulae, by the friction of the fluids on the inside surface of the tube during their flowing movement.

The deformation of the interface modifies the average optical properties, along the tube, of the plug obtained after said flowing. In other words, the average of an optical property, measured in a transversal cross-section, varies depending on the position of said transversal cross-section along the tube.

In particular, it is possible to use first and second fluids with different refractive indexes, so as to obtain a determined radial light emission profile. Advantageously, a method of the invention may therefore be used to make diffuser tips.

In a first main embodiment, the invention provides a method for manufacturing a plug, comprising the following steps:
a) filling, at least in part, a tube with a first fluid and a second fluid to create a fluid composite plug, said first and second fluids presenting different optical properties;
b) generating a differential pressure between the upstream surface and the downstream surface of said fluid composite plug, so as to deform the interface between the first fluid and the second fluid and get a distorted fluid plug;
c) optionally, curing said distorted fluid plug.

The fluid composite plug flows in the tube under the action of the differential pressure.

The first main embodiment is very well suited when it is possible to insert the first and second fluids in the tube before moving the fluid composite plug. For instance, the first and second fluids may be disposed inside the tube with a syringe.

The first main embodiment confers much flexibility. In particular, it is possible, at step a), to insert in the tubes several fluids, with adapted quantities and chosen optical properties, so as to easily create specific continuous variations of the average optical properties of the plug along the tube.

In a preferred second main embodiment, the invention provides a method for manufacturing a plug, comprising the following steps:
a') filling, at least in part, a tube with a first fluid, so that said first fluid flushes an opening of said tube, i.e. so that the first fluid substantially extends up to said opening;
b') plunging said opening of said tube into a second fluid preferably presenting different optical properties than said first fluid, so that said first and second fluids are in contact along an interface and sucking up said second fluid into said tube, so as to deform said interface and get a distorted fluid plug;
c') optionally, curing said distorted fluid plug.

The second main embodiment is advantageously very simple.

Contrary to the first main embodiment, no fluid composite plug is constituted inside the tube before deforming the interface. In the preferred embodiment, the interface between the two fluids is created when the tube, in which the first fluid has been previously sucked, is plunged into a reservoir of the second fluid. The interface then lies at the opening of the tube and is deformed while the second fluid is being sucked into the tube. Because of the friction on the inside surface of the tube, the fluids flow more rapidly around the axis of the tube and, consequently, the second fluid penetrates inside the first fluid. In fact, the second fluid does not touch the inside surface of the tube, but around said opening.

In some embodiments, during step b) or step b'), the tube may be inclined relatively to the horizontal direction and/or, during step b), a differential pressure may be generated so that said fluid composite plug moves upwards.

In an embodiment, the tube may be in an optically transparent material and/or have an inside diameter less than 1.5 mm, or less than 0.8 mm.

The invention also concerns a diffuser tip manufactured according to a method of the invention.

More generally, the invention concerns a diffuser tip comprising a first region made of a first material and a second region made of a second material, said first and second regions being separated by an interface, such that, in a median longitudinal cross-section, the line representing said interface, or "interface line", is at least partly, or completely, curved, i.e. is not only constituted by straight lines. This diffuser tip may comprise one or several of the essential or optional characteristics of a diffuser tip manufactured according to a method of the invention.

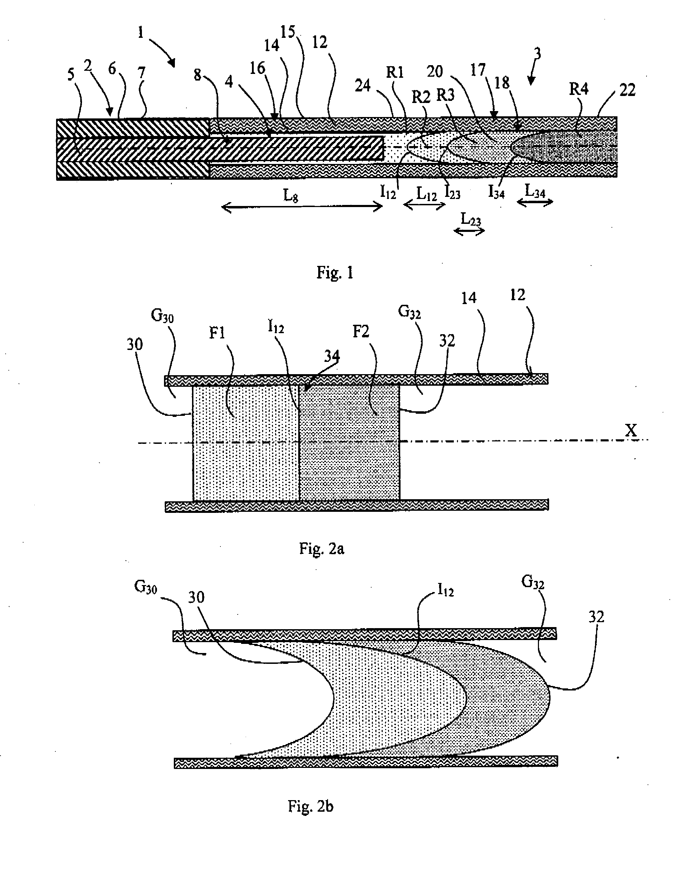

This diffuser tip may present one or several of the following optional characteristics:
- The interface line may be rounded or convex, in particular in the direction of the proximal end of the diffuser tip, as represented in figure 1. Preferably, the interface line remains convex over at least 10%, preferably over at least 50%, of its length, i.e. the interface line "turns" in the same direction when following at least 10% or 50% of its length. The interface line may be substantially parabolic.
- The interface line may be symmetric around the axis of the diffuser tip. It may be smooth, i.e. substantially without any irregularities.
- Said interface may be symmetric around the axis of the diffuser tip. This symmetry may be a rotational symmetry around the axis of the diffuser tip, i.e. so that the interface line has the same form in any median longitudinal cross-section.
- Said interface may be as long as necessary. In particular, it may have a length, along the axis of the diffuser tip, which is at least 10 mm and less than 70 mm.
- In an embodiment, none of the first and second regions is the core of an optical fibre, or is made of a transparent material which may be used for the core of an optical fibre.
- The volume of at least one of said regions may be more than 1 ml and less than 20 ml.
- At least one of said regions may comprise scattering centres. The concentration of scattering centres in a region may be more than 0.01 mg and less than 1.5 mg per gram of the material of said region. On average in a transversal cross-section, the concentration of scattering centres may continuously increase from one end of the diffuser tip towards the opposite end. In particular, it may increase when moving towards the distal end of the diffusing tip. Any profile of concentration of scattering centres, for instance in the form of a bell, is contemplated.
- The diffuser tip may comprise more than 2 and less then 6 regions separated by curved interfaces. However, the number of regions is not limited.
- The tube may have an inside diameter less than 1.5 mm.

The invention also concerns an optical device comprising an optical guide, in particular an optical fibre, and a diffuser tip according to the invention optically coupled with said optical guide, for instance with a distal end of an optical fibre. This optical device may also comprise a source of radiation presenting a power of more than 3 W, or a power of more than 8 W.

The invention also concerns a non-therapeutic or a therapeutic method to lighten, heat, irradiate an object, said method using an optical device of the invention.
The object may in particular be a cell, for instance a liver cell, or a tumour cell.

### DEFINITIONS

A "median longitudinal cross-section" of a diffuser tip is a cross-section along a plane including the axis of said diffuser tip.

A "transversal cross-section" of a diffuser tip is a cross-section along a plane perpendicular to the axis of said diffuser tip.

A "radial emission profile" represents the intensity of the radiation, in particular of light, which is radially emitted by a diffuser tip, in average in a transversal cross-section, as a function of the position of said transversal cross-section along the axis of the diffuser tip.

A "plug" is a mass which obstructs a tube, or a structural mass which previously obstructed a tube and has been extracted from said tube. For the sake of clarity, in a method according to the invention, the plug at the end of step a) is designated by "fluid composite plug" and the plug at the end of step b) is designated by "distorted fluid plug". A plug may have any shape.

A "structural mass" is a mass which does not flow, and may be solid or flexible.

### BRIEF DESCRIPTION OF THE DRAWINGS

Other characteristics and advantages of the present invention will be made apparent when reading the following description and examining the accompanying drawings in which:
- Figure 1 is a median longitudinal cross-section of an optical device manufactured according to the first main embodiment of the invention;
- Figures 2a and 2b are median longitudinal cross-sections of a tube in which two fluids have been inserted, before and after a step b), respectively;
- Figure 3 is a median longitudinal cross-section of a tube in which a viscous fluid flows;
- Figures 4 and 5 represent theoretical radial emission profiles of diffuser tips which may be manufactured according to the invention from 2 and 4 fluids, respectively;
- Figure 6 represents a step in which an optical fibre is being inserted into a tube containing a fluid composite plug made according to steps a) and b);
- Figure 7 represents three radial emission profiles of diffuser tips of the example, optically coupled with three optical fibres, with a radiation wavelengths of 804 nm;
- Figure 8 is a median longitudinal cross-section of an optical device manufactured according to a second main embodiment of the invention; and
- Figures 9a, 9b, and 9c represent successive operations of a method according to a second main embodiment of the invention.

These figures are for illustrative purpose only and are not limitating the scope of the invention.

The same references are used to designate same or similar members or parts in the different figures.

### DETAILED DESCRIPTION

FIG. 1 represents an optical device 1 in a first embodiment of the invention. This optical device 1 extends along an axis X and comprises an optical fibre 2 and a diffuser tip 3, optically coupled with a distal end 4 of the optical fibre 2.

The optical fibre 2 comprises an optically transmitive core 5, a reflective cladding 6, surrounding the core 5, and an outer jacket 7 which facilitates holding.

At its distal end 4, the cladding 6 and the outer jacket 7 have been removed, so that an uncladded portion 8 of the core 5 is penetrating axially into the diffuser tip 3.

The length L₈ of the uncladded portion 8 of the optical fibre 2 may be more than 0.5 cm, more than 1.0 cm, more than 2.0 cm, more than 3.0 cm or more than 5.0 cm and/or less than 10 cm. The longer the length of the uncladded portion 8 is, the better the adhesion of the diffuser tip 3 on the optical fibre 2 is. A length of 40 mm is well adapted.

The diffuser tip 3 comprises a transparent tube 12 defining coaxial cylindrical inside surface 14 and outside surface 15. A proximal portion 16 of the tube 12 receives the uncladded portion 8 of the core 5 of the optical fibre 2, whereas a distal portion 17 of the tube 12 is filled with a plug 18.

The plug 18 comprises four regions R1, R2, R3 and R4 presenting different concentrations C1, C2, C3, and C4 of TiO₂ scattering centres 20, respectively, the concentration of scattering centres increasing when approaching the distal end 22 of the tube 12. For instance, C1 = 0.02 mg/g; C2 = 0.04 mg/g; C3 = 0.13 mg/g; and C4 = 0.89 mg/g.

The four regions R1, R2, R3 and R4 are interpenetrating each other, so that, on average in a transversal cross-section, the concentration of scattering centres 20 continuously increases from the proximal end 24 of the distal portion 17 of the tube 12 to the distal end 22 of the tube 12.

The length L₁₂ of the interface I₁₂ between the regions R1 and R2, along the axis X, is about 40 mm. The length L₂₃ of the interface I₂₃ between the regions R2 and R3, along the axis X, is about 20 mm. The length L₃₄ of the interface I₃₄ between the regions R3 and R4, along the axis X, is about 10 mm. Generally, for a tip diffuser according to the invention, an interface between two adjacent regions may have a length which is at least 10 mm, at least 20 mm, at least 30 mm, at least 35 mm and/or less than 70 mm, less than 60 mm, less than 50 mm or less than 45 mm, for instance.

In the median longitudinal cross-section of FIG. 1, the interfaces are represented by curved parabolic interface lines.

Such a diffuser tip may be made according to a method comprising the above-mentioned steps a), b) and c).

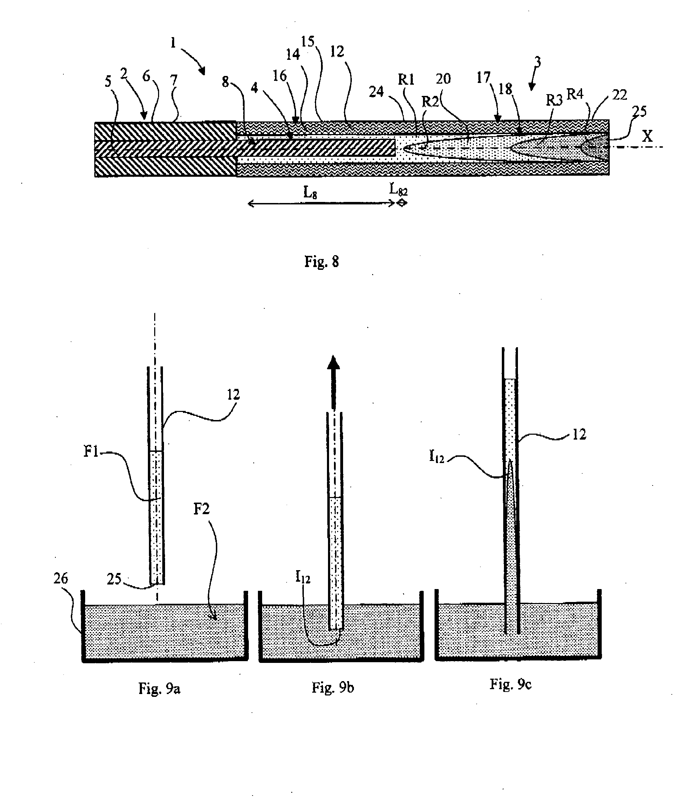

FIG. 8 represents an optical device 1 in a second embodiment of the invention. This optical device is similar to the optical device of FIG. 1, but has been made according to a method comprising the above-mentioned steps a'), b') and c'). Contrary to the embodiment of FIG.1, all the regions R1, R2, R3 and R4 extend up to the opening 25 through which the corresponding precursor fluids have been introduced into the tube 12. These regions R1-R4 are substantially only in contact with the inside surface of the tube at the opening 25 of said tube.

Region R2 is deeply penetrating into region R1. The same goes for region R3 into region R2, and region R4 into region R3. Preferably, the distance L₈₂ between the free end of the uncladded portion 8 of the optical fibre and region R2 is less than 10 mm, preferably less than 5 mm.

An example of a method comprising the above-mentioned steps a), b) and c) is now described in detail. However, this detailed description is not limiting the invention. In particular, the method could be run with any number of fluids.

### Step a)

At step a), the filling of the tube with the fluids may be achieved by suction or by injection. Successively sucking the different fluids into the tube advantageously limits the risk of undesired mixing of the fluids. For instance, the tube may be connected to a syringe and successively dipped into various reservoirs containing the different fluids so as to suck them into the tube.

For instance, as represented in FIG. 2a, a first fluid F₁ and a second fluid F₂ may be inserted in the tube to constitute a fluid composite plug 34. The interface I₁₂ between the first fluid F₁ and the second fluid F₂, the upstream surface 30, and the downstream surface 32 of said fluid composite plug 34 may be substantially plane and perpendicular to the axis X of the tube at the end of step a). Gel G₃₀ and G₃₂ may also be inserted into the tube, upstream and downstream the fluid composite plug, in contact with the upstream surface 30 and the downstream surface 32, respectively.

### FLUIDS

Preferably at least one, or preferably all, of the fluids filled in the tube are transparent fluids optionally containing scattering centres, or are able to be transformed into transparent materials optionally containing scattering centres, for example through curing.

At least one, or all, of the fluids filled in the tube at step a) may be silicon elastomers, and in particular RHODORSIL® RTV, 141A and B, sold by the RHODIA Company (France).

Advantageously, the polymerisation of RHODORSIL® leads to a material which presents a very good resistance to temperatures as high as 150°C or even 200°C.

The number of fluids in the tube before step b) is not limited. It may in particular be 2 or more, 3 or more, 4 or more, 5 or more, or 6 or more. It may be less than 10, less than 8 or less than 7. The insertion of 4 fluids is well-adapted to manufacture diffuser tips.

The volume of each fluid inserted into the tube is not limited. It may be more than 1 ml, more than 2 ml, more than 3 ml, more than 5 ml, more than 10 ml, and/or less than 20 ml, less than 17 ml, less than 15 ml, or less than 13 ml.

### SCATIERING CENTRES

At least one of the fluids may contain scattering centres, for instance made of alumina (Al₂O₃) and/or titania (TiO₂).

Titania is particularly advantageous because of its high reflexion index and chemical inertness.

The concentration "C" of scattering centres in a fluid may be more than 0.01 mg, more than 0.02 mg, more than 0.05 mg, more than 0.10 mg, more than 0.5 mg, more than 0.8 mg and/or less than 1.5 mg, less than 1.3 mg, less than 1.1 mg, or less than 1.0 mg per gram of fluid.

The concentration of scattering centres in a fluid may be uniform throughout a given fluid. In an embodiment, this concentration may also vary depending on the considered region of the fluid.

The particle size of the scattering centres may be less than 100µm.

The median size is preferably more than 2µm, more than 5 µm and/or less than 20µm, less than 15µm or less than 12µm. A median size of 10 µm is adapted.

### TUBE

For a diffuser tip, the tube may advantageously be in an optically transparent material, in particular PTFE or any equivalent tube regarding temperature properties and translucence. It may be flexible and/or straight.

The tube may present a cylindrical outside surface and/or a cylindrical inside surface. Preferably, its wall thickness is constant in a transversal cross-section and preferably in any transversal cross-section. The tube may present, in a transversal cross-section, or preferably in any transversal cross-section along its length, a circular outer contour and/or a circular inner contour. For a diffuser tip, a cylindrical outside surface with a circular base is preferred. Also, a cylindrical inside surface with a circular base is preferred.

The inside diameter may be less than 1.5 mm, less than 1.2 mm, less than 1.0 mm, less than 0.8 mm, less than 0.7 mm or less than 0.6 mm, and/or more than 0.2 mm, more than 0.3 mm, more than 0.4 mm, or more than 0.5 mm. An inside diameter of 0.56 mm or 0.8 mm is well-adapted.

The inside and/or outside surface may also comprise a texture, for instance to provide an optical effect, for instance a ground glass effect.

In an embodiment, a coating layer may be provided on the inside surface of the tube to allow an unmolding of the plug out of the tube after it has been cured. On the contrary, in another embodiment, a coating layer may be applied so as to favour the adhesion of the plug on the inside surface of the tube. For instance, this may be useful when the tube is transparent and is to be used as a protective jacket of a diffuser tip.

### Step b)

At step b), the fluid composite plug is distorted inside the tube under the action of a differential pressure between the upstream and downstream surfaces 30 and 32 of the fluid composite plug. The differential pressure to be applied may be determined, by simple tests, so as to obtain the desired deformation of the interfaces between the different fluids.

Without being bound by a theory, the inventors explain this deformation by the Navier-Stokes equations.

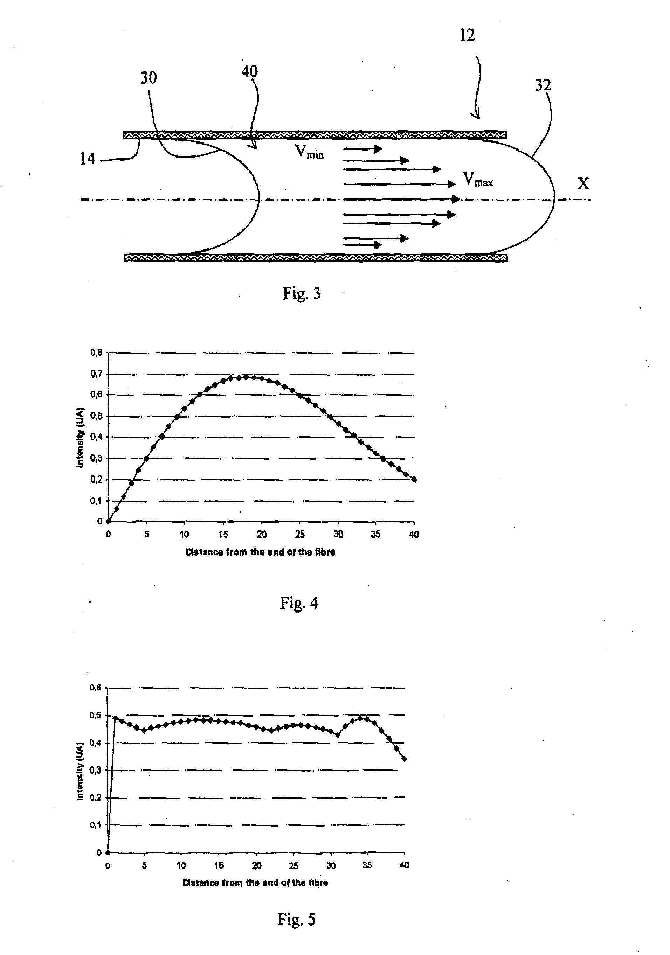

As it is represented in FIG. 3, and according to the Navier-Stokes equations, the speed of a region of a viscous fluid plug 40 in a tube 12, under the action of a differential pressure between the upstream surface 30 and the downstream surface 32 of said fluid plug, depends on the distance of said region from the inside surface 14 of the tube 12.

For example, in a tube presenting a circular cylindrical inside surface, the flow will be organized according to a parabolic speed field, with a minimal speed Vₘᵢₙ where the viscous fluid plug in contact with said inside surface 14 and a maximum speed Vₘₐₓ, around the axis X of the tube 12.

When the viscous fluid plug is a composite fluid plug, for example a composite fluid plug 34 comprising two fluids F1 and F2, as represented on FIG. 2b, the friction forces on the inside surface 14 of the tube 12 will therefore deform the interface(s) between the different fluids.

Hence, during step b), the different fluids are penetrating inside each other, preferably without mixing with each other. With such penetration, it is possible, using fluids with different diffusing properties, to obtain diffuser tips with specific radial emission profiles. For instance, the profile represented in FIG. 4 has the form of bell and could be obtained with 2 fluids. The profile represented in FIG. 5 could be obtained with 4 fluids. It shows that the emitted intensity along the axis X could be substantially constant along the diffuser tip.

The number of fluids, the volume of each fluid and its optical properties, and in particular the concentration of scattering centres, as well as the shape of the interfaces between the different fluids may be determined, by modelling, in particular according to the Navier-Stokes equations, and/or by routine tests, so as to obtain the desired radial emission profile.

During step b), the deformation of the interfaces between the fluids mainly depends on the natures of the fluids, the texture, the shape and the dimensions of the inside surface of the tube, the differential pressure and the temperature.

In particular, the inside surface of the tube may present ribs or grooves, in particular longitudinal or transversal ribs or grooves, obstacles, continuous or non continuous coating layer, one or several necks, so as to control the deformation of the interfaces between the fluids during step b).

For the same purpose, the tube may also be temporary bent or locally pressed during step b).

The differential pressure will push or pull the fluid composite plug inside the tube. It may be determined so that the maximum speed Vₘₐₓ of the fluid composite plug, usually around the axis of the tube, is less than 20 mm/s, preferably less than 10 mm/s.

Preferably, the difference of pressure is determined so as to maintain a laminar flow.

To generate the differential pressure, the fluid composite plug may for instance be submitted to an upstream pressure higher than the atmospheric pressure and/or to a downstream depression.

In an embodiment, during step b), the tube is inclined relatively to the horizontal direction, so as to benefit from the gravity force. In particular, the tube may be disposed vertically. However, to limit the generation of bubbles inside the fluid composite plug, it is preferable to suck the fluid composite plug so that it moves upwards.

The temperature may be the ambient temperature, i.e. around 20°C, or any other temperature, depending mainly on the natures of the fluids, and may be adapted to get the required viscosity of the fluids. As far as RHODORSIL® is concerned, steps a) and b) may be achieved at 20°C.

The temperature may be constant along the tube, or on the contrary, it may vary so as to locally modify the viscosities of the different fluids.

The fluid composite plug at the end of step b) is designated as a "distorted fluid plug". The optical properties of the distorted fluid plug, measured on average in a transversal cross-section, would depend on the position of this transversal cross-section along the axis X of the tube. Advantageously, these properties may continuously vary along this axis. Indeed, in a given transversal cross-section, the ratio of the area covered by a first fluid to the area covered by a second fluid will depend on the position of this transversal cross-section along the axis, at least in the part of the tube where the interface between these two fluids has been deformed.

As a variation of the invention, a second fluid is only used to deform the interface with a first fluid. At the end of step b), the second fluid, or at least part of this second fluid, is extracted out of the tube and replaced by a third fluid presenting useful properties in service, and in particular useful optical properties. Before the extraction of the second fluid, the first fluid may be cured to avoid any deformation of the interface during the extraction of the second fluid.

### Step c)

A method according to the invention may comprise, optionally, a step c) following step b), in which the distorted fluid plug is cured, for example through a polymerisation of the fluids. Step c) may comprise a heating treatment or an irradiation treatment, for instance.

When RHODORSIL® is used, the heating treatment may be operated at 50°C and may last, for instance, 5 hours.

The cured distorted plug may be solid or, preferably, flexible and elastic, for example as a silicon seal.

Depending on the intended application, the cured distorted plug may be extracted or not extracted out of the tube.

For a diffuser tip, it is preferable to use a tube which operates as a protective jacket for the diffuser tip. Advantageously, there is no need to extract the cured distorted plug out of the tube.

To make an optical device provided with a diffuser tip, the fluid distorted plug is preferably not cured before being preassembled with an optical guide, for instance an optical fibre. Before curing, the following operations are possible:

If necessary, the tube may first be cut to desired length. The distal end of the tube may then be closed, for instance with pliers, or any suitable closing means.

Preferably, the diffuser tip is configured so that all the radiation arriving at its proximal end is diffused laterally, i.e. without any return of radiation in the optical guide and without any excess of energy at the distal end of the diffuser tip. Advantageously, no reflector needs to be inserted at the distal end of the tube before closing it.

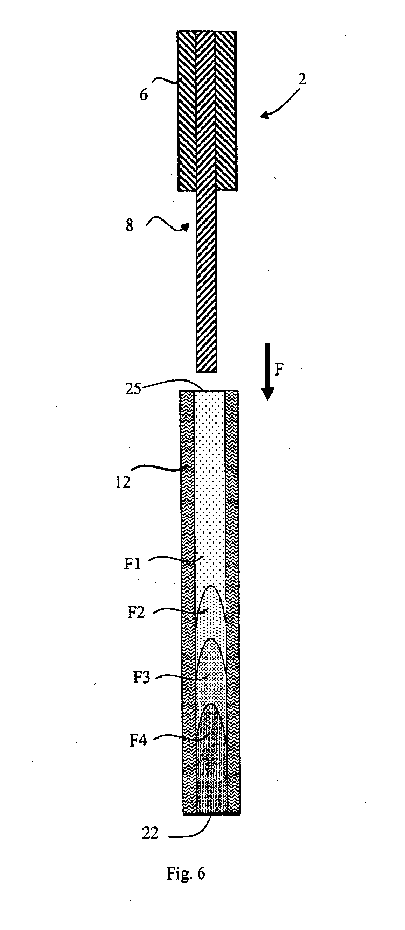

An uncladded portion of an optical fibre may then be inserted into the opening of the tube located at its proximal end, as represented in FIG. 6.

In FIG. 6, a tube 12 with a closed distal end 22 and an opened proximal end 25 is represented. This tube is filled, from the proximal end to the distal end, with fluids F1, F2, F3 and F4 containing increasing concentrations of scattering centres, the first fluid F1 flushing the opening of the proximal end of the tube 12. None of the fluids is cured. An uncladded portion 8 of an optical fibre 2 is introduced, axially, into the opening of the proximal end of the tube 12 (arrow F) until the cladding 6 abuts the tube 12. Preferably, the outer diameter of the diffusing tip is substantially the same as the outer diameter of the optical fibre, at least at the junction point, i.e. at the point where the tube abuts the cladding of the optical fibre.

The fluid distorted plug may then be cured, which will make the fluids adhere on the inside surface 14 of the tube 12, as well as on the uncladded portion 8 of the optical fibre.

Complementary steps are also contemplated. For instance, the diffuser tip may be shaped or polished.

An optical guide, in particular an optical fibre, provided with a diffuser tip of the invention is an optical device according to the invention. This optical device may comprise or be coupled with a source of radiation, in particular a source of light. This source of radiation may be configured so that the wavelength of that radiation is more than 630 nm and/or less than 800 nm.

However, the wavelength of the radiation is not limited, and in particular it may range between 400 nm and 1200 nm, depending on the materials which are used. Wavelengths less than 400nm or more than 1200nm are also contemplated.

The power of the source of radiation may be more than 3, 5, 8 or even more than 10 watts. Indeed, a diffuser tip according to the invention, such as the diffuser tip represented in FIG. 1, is very resistant to high temperatures and powers.

An optical device according to the invention may comprise a catheter, a cooling system, an insertion catheter an endoscope, or a fluorescence measuring device.

Preferably, a diffuser tip of the invention is adapted so as to diffuse light only radially.

An operator may use an optical device of the invention according to the common practice.

For instance, for laser induced thermotherapy, the distal end of an optical fibre provided with a diffuser tip of the invention will be introduced into a human body so as to dispose the diffuser tip adjacent to the tumour to be treated. Light will then be injected at the proximal end of the optical fibre, and be guided up to the diffuser tip. It will then radiate radially, and destroy the tumour by thermal effect.

### Steps a'), b') and c')

The steps a'), b') and c') are similar to the steps a), b) and c). But said first and second fluids are not introduced into the tube before deformation of the interface. In fact, the interface is deformed at the same time as the second fluid is sucked up into the tube.

In particular, the first fluid F1 may be sucked into the tube 12, so that it extends up to the opening 25 at the end of the tube (FIG. 9a). This end of the tube 12 is then plunged into a reservoir 26 of the second fluid F2 (FIG. 9b). The interface I₁₂ between the first and second fluids is then extending along the opening 25 of the tube, as represented in figure 9b. The second fluid F2 is then sucked up into the tube 12. Its interface I₁₂ with the first fluid is deformed at the same time as the second fluid enters into the tube.

Because of the friction forces on the inside surface of the tube, the second fluid penetrates into the first fluid, i.e. it progresses into the tube much faster than the first fluid located at the proximity of the inside surface of the tube 12. Therefore, the interface I₁₂ between the two fluids remains in contact the inside surface of the tube at the proximity of the opening 25.

The suction force of the second fluid F2 is preferably determined so that the flowing of the two fluids in the tube 12 remains laminar.

Preferably, the second fluid is moved inside the tube, so that, after assembly with an optical fibre, as represented in FIG. 8, the distance L₂₈ between said second fluid F2 and the end of the core of the optical fibre is less than 5 mm and, preferably, more than 1 mm or more than 3 or 4 mm.

All the characteristics described for the first main embodiment may be applied to the second main embodiment, provided there are not incompatible with this second main embodiment.

### EXAMPLE

The following preferred example does not limitate the invention.

At step a), the following fluids were successively sucked into a cylindrical PTFE tube, the inside diameter of this tube was 0.56 mm.
- Fluid 1: 11.59 ml of RHODORSIL®, corresponding to a length of 22.5 mm of the tube, with a concentration of 0.02 mg titania particles per gram of fluid;
- Fluid 2: 4.38 ml of RHODORSIL®, corresponding to a length of 8.5 mm of the tube, with a concentration of 0.04 mg of titania particles per gram of fluid;
- Fluid 3: 2.32 ml of RHODORSIL®, corresponding to a length of 4.5 mm along the tube, with a concentration of 0.13 mg of titania particles per gram of fluid;
- Fluid 4: 2.32 ml of RHODORSIL®, corresponding to a length of 4.5 mm along the tube, with a concentration of 0.89 mg of titania particles per gram of fluid.

The fluid composite plug constituted by the four fluids therefore extended along 40 mm of the tube (22.5 + 8.5 + 4.5 + 4.5).

A depression was exerted at the opening of the proximal end of the tube, while the tube was maintained in the vertical position, so that the proximal end was at the upper side of the tube. The depression was realised using a 10ml seringue. Filling speed was 5mm/s.

The tube was then cut and its distal end was closed.

The distal end of an optical fibre was uncladded over a length of 4 cm. The exposed core of the optical fibre, presenting a diameter of 400 microns, was then plunged into the opening at the proximal end of the tube, as represented in FIG. 6, until the cladding of the optical fibre contacts the tube. The fluid in excess, overflowing out of the tube during the introduction of the distal portion of the optical fibre, was eliminated.

The tube was then heated at 50°C for a time sufficient for the fluids to cure.

The proximal end of the optical fibre was coupled to a source of light and laser light was injected in the optical fibre (frequency: continuous mode; power: 10 Watts). The radial emission profile was measured.

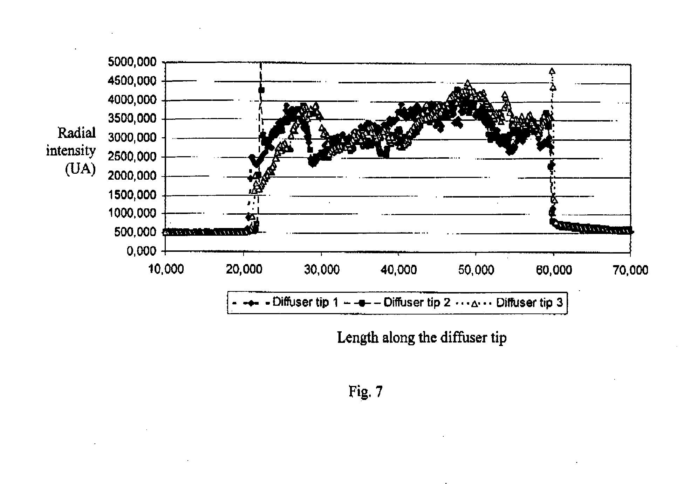

Three diffuser tips were manufactured as previously described and tested. FIG.7. represents the three radial emission profiles of these diffuser tips.

FIG.7. shows that the three diffuser tips have advantageously similar radial emission profiles. In this figure, the diffusion zone is also perfectly delimited. In addition, the intensity is substantially constant all along the diffuser tip. More precisely, this intensity does not deviate of more than +/- 20% from the average intensity in the diffusion zone.

It is now clear that the invention provides a solution to manufacture efficient diffuser tips, with a simple and flexible method.

Of course, the invention is not limited to the provided example or to the represented embodiments.

In particular, other optical guides than an optical fibre may be used.

Throughout the specification, including in the claims, the term "comprising a" should be understood as being synonymous with "comprising at least one" unless specifed to the contrary.

## Claims

1. A method for manufacturing a plug, comprising the flowing, in a tube (12), of a first fluid (F1) and a second fluid (F2), said second fluid (F2) being in contact, along an interface (I₁₂), with said first fluid (F1) so that the friction on the inside surface (14) of the tube (12) deforms said interface (I₁₂), said first and second fluids presenting different optical properties.

2. A method for manufacturing a plug according to claim 1, comprising the following steps:
a) filling, at least in part, a tube (12) with a first fluid (F1) and a second fluid (F2) to create a fluid composite plug (34), said first and second fluids presenting different optical properties;
b) generating a differential pressure between the upstream surface (30) and downstream surface (32) of said fluid composite plug (34), so as to deform the interface (I₁₂) between the first fluid and the second fluid and get a distorted fluid plug;
c) optionally, curing said distorted fluid plug.

3. A method for manufacturing a plug according to claim 1, comprising the following steps:
a') filling, at least in part, a tube (12) with a first fluid (F1), so that said first fluid (F1) substantially extends up to an opening (26) of said tube (12);
b') plunging said opening (26) of said tube (12) into a second fluid (F2) presenting different optical properties than said first fluid (F1), so that said first and second fluids are in contact along an interface (I₁₂), and sucking up said second fluid (F2) into said tube (12), so as to deform said interface (I₁₂) and get a distorted fluid plug;
c') optionally, curing said distorted fluid plug.

4. The method according to any one of the preceding claims, in which said first and second fluids present different refractive indexes.

5. The method according to any one of claims 2 and 3, in which, during step b) or b'), the tube (12) is inclined relatively to the horizontal direction.

6. The method according to any one of the preceding claims, in which the tube (12) is in an optically transparent material.

7. The method according to any one of the preceding claims, in which the tube (12) has an inside diameter less than 1.5 mm.

8. The method according to any one of the preceding claims, in which the fluids filled in the tube (12) are transparent fluids optionally containing scattering centres, or are able to be transformed into transparent materials optionally containing scattering centres.

9. A diffuser tip comprising a first region made of a first material (R1) and a second region (R2) made of a second material, said first and second regions being separated by an interface (I₁₂) such that, in a median longitudinal cross-section, the line representing said interface, or "interface line", is at least partly curved.

10. The diffuser tip according to the preceding claim, manufactured according to a method according to any one of claims 1 to 8.

11. The diffuser tip according to any one of claims 9 to 10, in which the volume of at least one of said regions is more than 1 ml and less than 20 ml.

12. The diffuser tip according to any one of claims 9 to 11, in which at least one of said regions comprises scattering centres (20).

13. The diffuser tip according to the previous claim, in which, on average in a transversal cross-section, the concentration of scattering centres (20) continuously increases from one end of the diffuser tip towards the opposite end.

14. The diffuser tip according to any one of claims 9 to 13, comprising more than 2 and less then 6 regions separated by curved interfaces.

15. An optical device comprising an optical guide (2), a diffuser tip (3) according to any one of claims 9 to 14 and optically coupled with said optical guide, and a source of radiation presenting a power of more than 8 W.
